# EUROPEAN PATENT SPECIFICATION

(11) **EP 4 291 270 B1**
(45) Date of publication and mention of the grant of the patent: **11.12.2024**
(21) Application number: 22707508.2
(22) Date of filing: 11.02.2022
(51) Int. Cl.: A61M 5/31, A61J 1/14, A61M 39/20

(54) **CAP FOR A SYRINGE WITH LUER-LOCK CONNECTOR**
KAPPE FÜR EINE SPRITZE MIT LUER-LOCK-ANSCHLUSS
CAPUCHON POUR UNE SERINGUE AVEC CONNECTEUR LUER-LOCK

(30) Priority: 15.02.2021 IT 202100003320
(43) Date of publication of application: 20.12.2023
(73) Proprietor: Platinum Pharma Service S.r.l., 65013 Città Sant'Angelo (PE) (IT)
(72) Inventor: RICCI, Alfredo, 65010 Cappelle sul Tavo (PE) (IT)
(74) Representative: Di Bernardo, Antonio
(86) International application number: PCT/IB2022/051234
(87) International publication number: WO 2022/172211

(56) References cited:
- EP-A1- 2 862 587
- US-A1- 2006 178 627
- US-A1- 2019 344 017

## Description

### TECHNICAL FIELD

The present invention relates, in general, to the technical field of syringe caps. More particularly, the present invention relates to an air-tight and unintentional anti-unscrewing closure cap for a disposable syringe with Luer-lock connector. The invention also relates to a syringe with Luer-lock connector provided with such a cap.

### BACKGROUND OF THE INVENTION

Syringes, in particular pre-filled ones, are widely used to inject medicinal products in a person such as, among others, anticoagulants, for example heparin and the like, vaccines, small sized molecules, and cosmetic products, for example hyaluronic acid. They represent a growing market, as they make it possible to effectively reduce errors in the dosage of a product to be injected and help to reduce packaging costs.

A syringe typically comprises a transparent cylindrical body, on which measurement notches are indicated, a plunger sliding inside the transparent cylindrical body and a hollow and frusto-conical tip, extending from the transparent cylindrical body and onto which a needle, it also being hollow, is subsequently fitted through which the product to be injected, contained in the hollow cylindrical body, passes. The plunger is provided, at one end thereof, with a rubber ring or capsule to ensure the controlled and constant sliding of the plunger inside the transparent cylindrical body. The tip of the syringe can be provided with a Luer-lock connector, i.e. a screwing system that locks the needle connection to prevent the unintentional removal thereof.

Syringes, particularly pre-filled ones, may also be provided with a closure cap, the function of which is to prevent the leakage of the product with which the syringe was previously filled, when the syringe is not in use.

With particular reference to pre-filled syringes, they must guarantee the sterility of the product inside them for several years, typically up to five years, i.e. they must prevent the product from coming into contact with air. In fact, if air enters the pre-filled syringe, the product inside it could undergo such deteriorations as to compromise the effectiveness thereof.

The entry of air into a pre-filled syringe typically occurs following repeated thermal cycles and/or unintentional impacts, which phenomena might cause the partial or total separation of the cap from the syringe. The entry of air into the pre-filled syringe might also occur during the syringe sterilization process, for example in an autoclave, or in the centrifugation phase, during which a pressure drop is generated in the syringe that promotes the entry of air.

The closure caps for the syringes, in particular for pre-filled syringes, with a Luer-lock connector must therefore have sealing properties, i.e. ensure airtightness, and anti-unscrewing properties, i.e. be configured so as not to separate unintentionally and in an unwanted manner from the syringe, when the syringe is not in use, typically during storage.

A closure cap for pre-filled syringe with Luer-lock connector of known type comprises a rigid and relatively non-deformable portion for handling and coupling with the Luer-lock connector, and a softer and hollow portion, typically made of rubber, which is housed in the rigid portion and is designed to come into contact, by wrapping it, with the frusto-conical tip of the syringe.

The known cap briefly described above has some drawbacks.

Firstly, this cap, even if correctly positioned at the tip and at the Luer-lock connector of the syringe, does not guarantee a sufficient sealing and unintentional anti-unscrewing action, as the contact force between the rubber portion and the tip of the syringe is distributed over a surface, namely the frusto-conical external side surface of the tip, which is relatively extended. The soft rubber portion of the cap therefore exerts a relatively low pressure on the external side surface of the tip, with consequent possibility of air entering the pre-filled syringe and simultaneous reduction of the anti-unscrewing effect exerted by the cap at the Luer-lock connector, under storage conditions of the pre-filled syringe. This anti-unscrewing effect is in fact due to the frictional forces generated between the external frusto-conical surface of the syringe tip and the soft rubber portion of the cap.

Secondly, when mounting the cap onto the tip of the pre-filled syringe, air can remain trapped between the soft rubber portion of the cap and the tip of the pre-filled syringe, which trapped air is inevitably forced into the pre-filled syringe, as the soft rubber portion is fitted onto the pre-filled syringe.

Furthermore, in the step of inserting the cap onto the pre-filled syringe, a creeping action of the cap is generated on the external side surface of the tip. This creeping, in addition to being annoying for the operator, can cause the generation of unwanted microparticles that might contaminate the product contained in the syringe.

Finally, the closure caps for syringes of the known type are difficult to assemble for the manufacturer and have a relatively high cost.

Patent documents US 2019/344017 A1, EP 2 862 587 A1 and US 2006/178627 A1 describe closure caps for syringes according to the prior art.

### SUMMARY OF THE INVENTION

The main object of the present invention is therefore to provide a cap for a syringe with Luer-lock connector capable of overcoming the drawbacks mentioned above with reference to the caps for a syringe of the known type.

More specifically, the main object of the present invention is to provide a cap for a syringe with Luer-lock connector configured in such a way as to considerably increase the sealing and unintentional anti-unscrewing properties, in particular during the step of the autoclave sterilization of the syringe, a step during which high pressure, humidity and temperature values are reached, so as to guarantee perfect preservation of the product contained in the syringe, at least for a period of five years.

Still another object of the present invention is to provide a cap for a syringe with Luer-lock connector configured to facilitate the screwing operation onto the syringe, as well as the unscrewing operation when the syringe is to be used to inject the product contained therein.

Not the least object of the present invention is to provide a cap for a syringe with Luer-lock connector, which can be produced in times and at costs lower than those for producing traditional caps.

These and other objects, which will become clearer in the following of the present description, are achieved by a cap for a syringe with Luer-lock connector and by a syringe according to claims 1 and 11. Preferred characteristics of the cap are disclosed in dependent claims 2 to 10.

The invention thus relates, in a first aspect thereof, to a cap of a syringe with Luer-lock connector comprising:
- a main body provided with a first handle portion and a second coupling portion having an external threading for coupling with a corresponding internal threading of the Luer-lock connector of the syringe, an axial cavity having an aperture at the second coupling portion being formed in the main body; and
- a rubber housed within the axial cavity of the main body, wherein the rubber has a surface facing the aperture of the axial cavity and designed to abut against an open free end of a tip of the syringe for air-tightly closing the open free end.

The cap is characterized in that raised elements are formed at respective crests of the threads of the external threading of the second coupling portion and in that the raised elements are dimensioned so as to frictionally couple with a bottom of respective threads of the internal threading of the Luer-lock connector.

In a second aspect thereof, the invention relates to a syringe, preferably a pre-filled syringe, with a Luer-lock connector provided with a cap as defined above.

When the cap is mounted onto the syringe, friction is generated between the raised elements provided on the external threading of the cap and the threading of the Luer-lock connector of the syringe and the rubber abuts against the tip of the syringe, suitably compressed inside of the axial cavity. The unintentional anti-unscrewing and air-tight closure of the syringe is thus ensured for long periods of time.

In other words, the sealing and anti-unscrewing characteristics of the cap according to the invention are ensured by two factors, namely the friction force generated between the raised elements of the cap and the internal threading of the Luer-lock connector of the syringe, a force which is added to the friction between the flanks of the threads of the external threading of the cap and the flanks of the threads of the internal threading of the Luer-lock connector, and the elastic reaction of the rubber, compressed inside the axial cavity. In fact, the rubber, in addition to air-tightly closing the tip of the syringe, increases, due to its elastic reaction, the frictional force between the second coupling portion of the main body and the Luer-lock connector to which the cap is coupled.

Yet, since the rubber abuts against the tip of the syringe and is constantly compressed inside the axial cavity of the main body, the elastic reaction of the rubber remains in an interval such as to guarantee the anti-unscrewing effect and the sealing power of the cap. It is therefore avoided that the rubber undergoes a plastic deformation over time, which would affect the efficiency of the tight closure of the free open end of the syringe tip.

### BRIEF DESCRIPTION OF THE DRAWINGS

Further characteristics and advantages of the present invention will become clearer from the following detailed description of a preferred embodiment thereof, given below, by way of non-limiting example, with reference to the attached drawings. In the drawings:
- Figure 1 is a perspective view of a cap for a syringe with Luer-lock connector according to a preferred embodiment of the present invention;
- Figure 2 is an exploded perspective view of the cap of Figure 1 and part of a Luer-lock connector of a syringe;
- Figure 3 is a perspective and longitudinal section view of the cap of Figure 2 and part of a Luer-lock connector of a syringe;
- Figure 4 is an enlarged perspective view of a detail of a coupling portion of the cap of Figure 1;
- Figure 5 is a partial perspective view and in longitudinal section of the cap of Figure 1, mounted on a syringe with Luer-lock connector;
- Figure 6 is an enlarged view of a detail of Figure 5; and
- Figure 7 is a perspective view of the cap of Figure 1 mounted on a syringe with Luer-lock connector.

### DETAILED DESCRIPTION OF PREFERRED EMBODIMENTS OF THE INVENTION

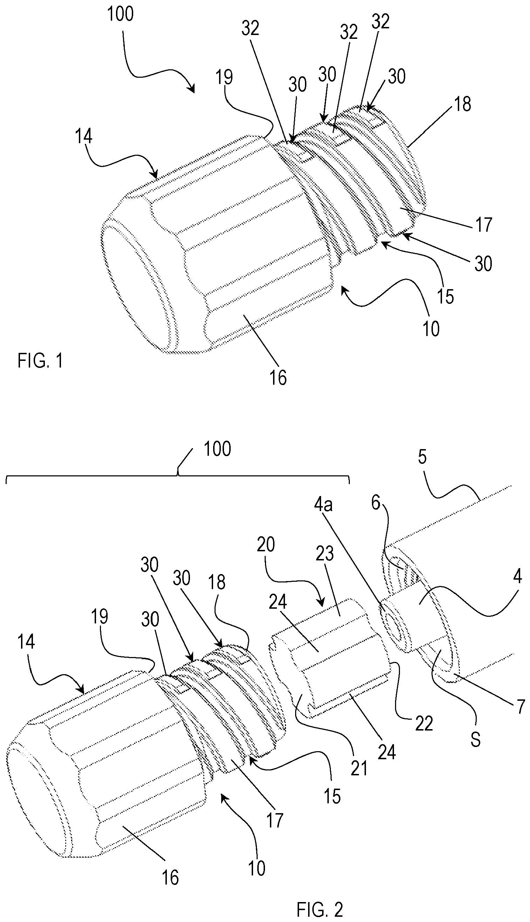

With reference to Figures 1 to 6, a cap for a syringe with Luer-lock connector according to a preferred embodiment of the present invention is illustrated.

The cap, generally indicated with the reference number 100, comprises a main body 10, substantially cylindrical, including a first portion 14 for handling the cap 100 and a second portion 15 for coupling the cap 100 with a syringe with Luer-lock connector.

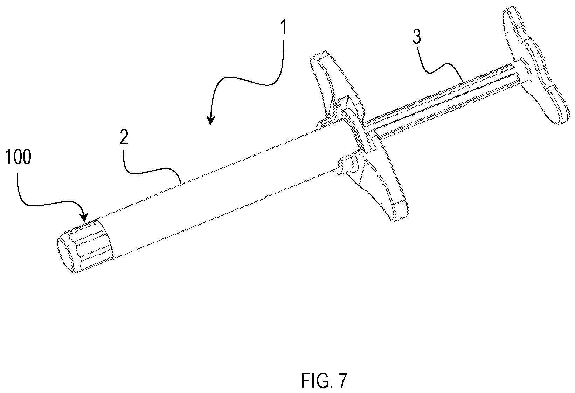

As shown in detail in Figures 2, 3 and 7, the syringe, generally indicated by reference number 1, is preferably a pre-filled syringe and comprises a typically transparent cylindrical body 2, for containing a product to be injected, and a plunger 3 sliding inside the transparent cylindrical body 2. A hollow and substantially frusto-conical tip 4, onto which a needle (not shown) is subsequently fitted for the injection of the product present in the transparent cylindrical body 2, extends from the transparent cylindrical body 2. At the tip 4 there is a Luer-lock connector 5 provided with an internal threading 6. A space S is delimited between the Luer-lock connector 5 and the tip 4.

The first handle portion 14 preferably has a faceted external surface 16 to facilitate the gripping of the cap 100 by a user in the step of screwing/unscrewing the cap 100 onto/from the syringe 1.

Preferably, the second coupling portion 15 has an external diameter smaller than the external diameter of the first handle portion 14, so that an annular surface 19 is delimited between the first handle portion 14 and the second coupling portion 15, which is designed to abut against a corresponding annular surface 7 of the Luer-lock connector 5, when the cap 100 is mounted on the syringe 1.

The second coupling portion 15 also has a conical mouth 18 at a free end thereof, which advantageously facilitates the insertion of the cap 100 into the space S comprised between the tip 4 and the Luer-lock connector 5 of the syringe 1.

The second coupling portion 15 has, in addition, an external threading 17 for coupling with the internal threading 6 of the Luer-lock connector 5 of the syringe 1.

In the external threading 17 of the second coupling portion 15, preferably at a crest portion of the threads, raised elements 30 are formed, preferably having a substantially rectangular or trapezoidal section, the function of which is to generate friction with corresponding threads of the internal threading 6 of the Luer-lock connector 5 in order to ensure a tight and unintentional anti-unscrewing closure of the cap 100 on the syringe 1.

In the illustrated embodiment, two sets of raised elements 30 are provided, each comprising at least one raised element, preferably three raised elements 30, provided on the external threading 17 in a diametrically opposite position. Alternatively, only one set of raised elements may be provided, or a number of sets of raised elements 30 greater than two, suitably distributed on the external threading 17 along the circumference of the second coupling portion 15, each set being composed of a number of raised elements less than or greater than three. For example, if three sets of raised elements are provided, these sets are preferably arranged at 120 degrees around the circumference of the second coupling portion 15 of the cap 100.

Preferably, the raised elements 30 of each set of raised elements are aligned with each other along the external threading 17 of the second coupling portion 15 of the cap 100 according to a longitudinal axis of the cap 100.

Preferably, the raised elements 30 are recessed or aligned, but never protruding, with respect to an edge of the external threading 17 of the second coupling portion 15 of the cap 100.

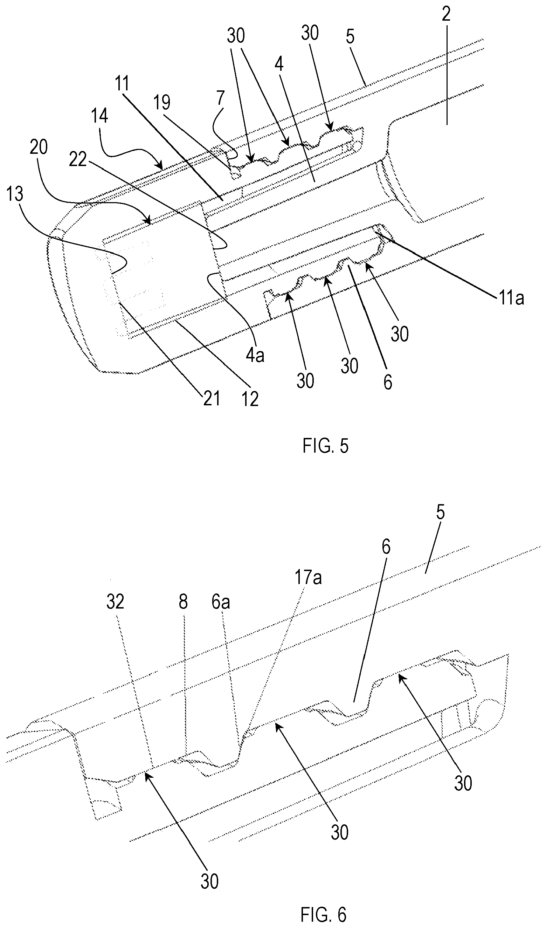

In particular, and as shown in detail in Figure 6, the raised elements 30 constitute active components, which cause, when screwing the cap 100 onto the Luer-lock connector 5 of the syringe 1, a localised static friction to be generated between an upper surface 32 of the raised elements 30 and a bottom 8 of respective threads of the internal threading 6 of the Luer-lock connector 5.

This localised static friction is added to the one generated between the flanks 6a and 17a, respectively, of the internal threading 6 of the Luer-lock connector and the external threading 17 of the cap 100, advantageously avoiding that, in particularly extreme conditions, for example during the step of sterilisation of the syringe 1 in an autoclave, where high values of pressure, humidity and temperature are reached, a loosening of the cap 100 occurs, with consequent reduced tightness of the same and penetration of air into the cylindrical body of the syringe 1.

In fact, this configuration of the external threading 17 of the second coupling portion 15, in particular the presence of the raised elements 30, ensures that the interference between the cap 100 and the Luer-lock connector 5 is contained in a well-defined region, thus obtaining an effective unintentional anti-unscrewing effect and, at the same time, allowing the Luer-lock connector to deform elastically just enough to avoid cracks and breakage.

Preferably, the threads of the cap 100 and the threads of the Luer-lock connector 5 are slightly tapered, thereby further facilitating the operations of screwing and unscrewing the cap 100 on the syringe 1. In fact, the contact forces between the cap 100 and the Luer-lock connector 5 do not persist for the entire duration of the screwing and unscrewing steps of the cap - that is, they are present in the final part of the screwing and in the initial part of the unscrewing. Furthermore, the contact forces between cap 100 and Luer-lock connector 5 are also generated at the raised elements 30.

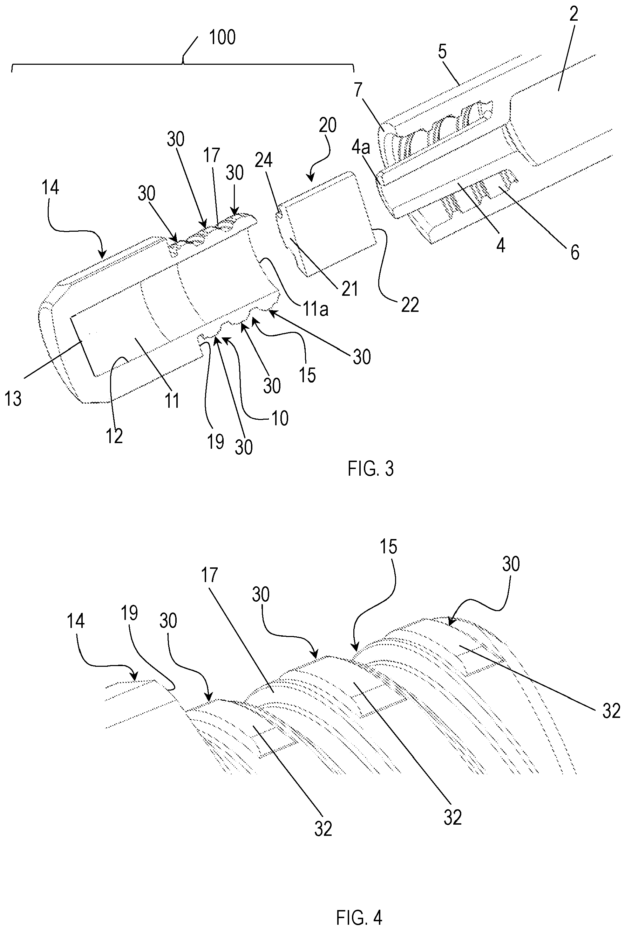

As clearly visible in Figure 3, an axial cavity 11, it being also substantially cylindrical, having an aperture 11a at the second coupling portion 15 is formed in the main body 10. The axial cylindrical cavity 11 also has a side surface 12 and a plane bottom surface 13, perpendicular to the longitudinal axis of the cap 100.

The main body 10 is made of a rigid and non-deformable material, for example compact polycarbonate (Makrolon^{®}), cyclic-olefin polymer (COP), or cyclic-olefin copolymer (COC), and the plane bottom surface 13 of the axial cylindrical cavity 11 is also rigid and non-deformable.

The cap 100 also comprises a rubber 20, which is housed in the axial cylindrical cavity 11 of the main body 10.

The rubber 20 is substantially cylindrical in shape and has a surface 21, a surface 22, opposite to the surface 21 and facing the aperture 11a of the axial cavity 11 so as to abut, in use, against a free end 4a of the tip 4 of the syringe 1, and a side surface 23.

The surfaces 21 and 22 are preferably plane, while, at the side surface 23, at least one longitudinal groove 24, preferably a pair of opposed longitudinal grooves 24, more preferably a series of longitudinal grooves 24 are formed, which allow the user to deform the rubber 20 to advantageously facilitate the insertion thereof into the axial cylindrical cavity 11 of the main body 10, during the assembly of the cap 100. The longitudinal grooves 24 also advantageously allow air to escape when inserting the rubber 20 into the axial cavity 11 during the assembly phase of the cap 100, so that no air is trapped between the bottom surface 13 and the surface 21 of the rubber 20.

Alternatively, instead of being plane, the surface 22 of the rubber 20 designed to abut, in use, against the free end 4a of the tip 4 of the syringe 1 can be convex, with convexity facing the tip 4. In this case, as the cap is screwed onto the syringe, the convex surface 22 of the rubber 20 flattens, thus abutting against the free end 4a of the syringe 1, air-tightly closing the opening thereof.

In the assembled condition of the cap 100, shown in detail in Figure 5, the surface 21 of the rubber 20 abuts against the bottom surface 13 of the axial cylindrical cavity 11 of the main body 10 and the side surface 23 - visible in Figure 2 - is in contact with the side surface 12 of the internal axial cylindrical cavity 11 of the main body 10. On the other hand, the surface 22 of the rubber 20 abuts against the free end 4a of the tip 4 of the syringe 1.

In the illustrated embodiment, the bottom surface 13 of the axial cylindrical cavity 11 is a plane, rigid surface. Alternatively, recesses may be formed at the bottom surface 13, between which an abutment surface 21 of the rubber 20 is delimited.

In yet another alternative embodiment, a seat for housing a pin extending from the bottom surface 13 of the axial cylindrical cavity 11 of the main body 10 may be formed in the surface 21 of the rubber 20.

With reference to Figures 5 and 6, the sealing and unintentional anti-unscrewing action of the cap according to the invention is described below when the cap is mounted on the syringe 1.

In the mounting condition of the cap 100 on the syringe 1, the second coupling portion 15 of the main body 10 is screwed onto the Luer-lock connector 5 by coupling the corresponding threadings 17 and 6.

In particular, each raised element 30 formed on the external threading 17 of the second coupling portion 15 is frictionally coupled with the bottom 8 of a corresponding thread of the internal threading 6 of the Luer-lock connector 5 and this localised static friction is added to that produced by the coupling between flanks 6a and 17a, respectively, of the internal threading 6 of the Luer-lock connector and of the external threading 17 of the cap 100.

The advantage given by the frictional coupling between each raised element 30 and the bottom 8 of a corresponding thread of the threading of the Luer-lock connector is that it does not have such a tangential component as to unscrew the cap. The tangential component is instead always present in the friction between the flanks of the threads, since the threads are helical, and therefore the friction tangent to the flanks of the threads has a component that tends to unscrew the cap from the syringe. This aspect is of fundamental importance when the syringe, e.g. pre-filled with acid and placed in an autoclave, is subject to internal pressure. Indeed, the internal pressure pushes the cap axially and the tangential component of the friction between the flanks of the threads would tend to unscrew the cap. However, thanks to the friction between each raised element 30 and the bottom 8 of a corresponding thread of the threading of the Luer-lock connector 5, which has no such a tangential component as to unscrew the cap, the cap is prevented from unscrewing from the syringe 1.

Furthermore, the annular surface 19 of the cap 100 abuts against the corresponding annular surface 7 of the Luer-lock connector 5, thus providing a mechanical stop and at the same time ensuring a suitable elastic deformation of the rubber 20. In fact, when the annular surface 19 is in abutment against the Luer-lock connector 5, the rubber 20 has the plane surface 22 in abutment against the free end 4a of the tip 4 of the syringe 1 and the surface 21 against the plane, rigid bottom surface 13 of the axial cylindrical cavity 11 of the main body 10. The rubber 20 is therefore suitably elastically compressed between the free end 4a of the syringe 1 and the bottom surface 13 of the main body 10 of the cap 100.

Under its compressed condition, the rubber 20 generates an appropriate elastic reaction force, which acts on the threaded coupling between the second coupling portion 15 and the Luer-lock connector 5, so that the threadings 6 and 17 of the Luer-lock connector 5 and of the cap 100 press between them, completely eliminating any clearance between the threads, generating friction between them and therefore eliminating any possibility of unintentional unscrewing of the cap 100 from the syringe 1.

Furthermore, since the contact between rubber 20 and tip 4 of the syringe 1 is essentially frontal, that is, between the plane surface 22 of the rubber 20 and the free end 4a of the tip 4 of the syringe 1, there is no friction between the rubber 20 and the external surface of the tip 4. It follows that the operations of screwing and unscrewing the cap 100 by the operator are facilitated.

Under this mounted condition, the cap according to the invention achieves the desired air-tight and anti-unscrewing effect.

In fact, the rubber 20, in addition to air-tightly closing the free end 4a of the tip 4 of the syringe 1, due to the effect of the elastic deformation, increases the degree of coupling between the threadings 6 and 17, of the Luer-lock connector 5 and of the second coupling portion 15, respectively, with an anti-unscrewing effect which is very similar to that obtained by inserting an elastic washer between screw and nut, tightening the latter.

The localised static friction force generated between the surface 32 of each raised element 30 of the external threading 17 of the second coupling portion and the bottom 8 of the corresponding thread of the internal threading 6 also contributes to increase the anti-unscrewing effect of the Luer-lock connector 5 of the syringe 1.

Furthermore, the presence of the rubber 20 eliminates the anti-unscrewing effects due to thermal cycles and/or vibrations since the elastic material is able to absorb these effects, always guaranteeing an adequate elastic reaction force.

From the above description, the characteristics of the cap for a syringe with Luer-lock connector, and of the syringe comprising it, of the present invention are clear, as well as are the relative advantages.

Finally, it is clear that the cap as conceived herein is susceptible to many modifications and variations. In practice, the materials used, as well as their dimensions, can be of any type according to the technical requirements. For example, the cap and the cylindrical body of the syringe can be made of the same material, so as to advantageously have the same thermal expansion coefficient.

## Claims

1. A cap (100) for a syringe (1) with Luer-lock connector (5) comprising:
- a main body (10) provided with a first handle portion (14) and a second coupling portion (15) having an external threading (17) for coupling with a corresponding internal threading (6) of the Luer-lock connector (5) of the syringe (1), an axial cavity (11) having an aperture (11a) at the second coupling portion (15) being formed in the main body (10); and
- a rubber (20) housed in the axial cavity (11) of the main body (10), wherein the rubber (20) has a surface (22) facing the aperture (11a) of the axial cavity (11) and designed to abut against an open free end (4a) of a tip (4) of the syringe (1) for air-tightly closing the open free end (4a);
**characterized in that** raised elements (30) are formed at respective crests of threads of the external threading (17) of the second coupling portion (15) and **in that** the raised elements (30) are dimensioned so as to frictionally couple with a bottom (8) of corresponding threads of the internal threading (6) of the Luer-lock connector (5).

2. A cap (100) according to claim 1, wherein the raised elements comprise at least two sets of raised elements (30) provided in a diametrically opposite position on the external threading (17).

3. A cap (100) according to claim 2, wherein each set of raised elements comprises at least one raised element (30).

4. A cap (100) according to claim 2 or 3, wherein the raised elements (30) of each set of raised elements are aligned therebetween along the external threading (17) of the second coupling portion (15).

5. A cap (100) according to any one of the preceding claims, wherein the raised elements (30) have a substantially trapezoidal or rectangular section.

6. A cap (100) according to any one of the preceding claims, wherein the raised elements (30) are recessed or aligned with respect to an edge of the external threading (17) of the second coupling portion (15).

7. A cap (100) according to any one of the preceding claims, wherein the surface (22) of the rubber (20) facing the aperture (11a) of the axial cavity (11) is plane or convex with convexity facing the aperture (11a) of the axial cavity (11).

8. A cap (100) according to any one of the preceding claims, wherein the axial cavity (11) comprises a side surface (12) and a rigid bottom surface (13), against which a surface (21) of the rubber (20) opposite to the surface (22) facing the aperture (11a) of the axial cavity (11) abuts.

9. A cap (100) according to any one of the preceding claims, wherein the rubber (20) further comprises at least one longitudinal groove (24) formed in a side surface (23) thereof.

10. A cap (100) according to any one of the preceding claims, wherein the second coupling portion (15) has a conical mouth (18) at a free end thereof.

11. A syringe (1) with Luer-lock connector (5), preferably a pre-filled syringe comprising a cap (100) according to any one of the preceding claims.

## Patentansprüche

1. Kappe (100) für eine Spritze (1) mit Luer-Lock-Anschluss (5), umfassend:
- einen Hauptkörper (10), der mit einem ersten Griffabschnitt (14) und einem zweiten Kupplungsabschnitt (15) bereitgestellt ist, der ein Außengewinde (17) zum Kuppeln mit einem entsprechenden Innengewinde (6) des Luer-Lock-Anschlusses (5) der Spritze (1) aufweist, wobei in dem Hauptkörper (10) ein axialer Hohlraum (11), der eine Öffnung (11a) an dem zweiten Kupplungsabschnitt (15) aufweist, ausgebildet ist; und
- einen Gummi (20), der in dem axialen Hohlraum (11) des Hauptkörpers (10) untergebracht ist, wobei der Gummi (20) eine Oberfläche (22) aufweist, die der Öffnung (11a) des axialen Hohlraums (11) zugewandt ist und dafür ausgelegt ist, an einem offenen freien Ende (4a) einer Spitze (4) der Spritze (1) anzuliegen, um das offene freie Ende (4a) luftdicht zu verschließen;
**dadurch gekennzeichnet, dass** hervorstehende Elemente (30) an entsprechenden Scheiteln von Gewinden des Außengewindes (17) des zweiten Kupplungsabschnitts (15) ausgebildet sind und dass die hervorstehenden Elemente (30) so dimensioniert sind, dass sie mit einem Boden (8) entsprechender Gewinde des Innengewindes (6) des Luer-Lock-Verbinders (5) reibschlüssig verbunden sind.

2. Kappe (100) gemäß Anspruch 1, wobei die hervorstehenden Elemente mindestens zwei Sätze von hervorstehenden Elementen (30) umfassen, die in einer diametral gegenüberliegenden Position auf dem Außengewinde (17) bereitgestellt sind.

3. Kappe (100) gemäß Anspruch 2, wobei jeder Satz von hervorstehenden Elementen mindestens ein hervorstehendes Element (30) umfasst.

4. Kappe (100) gemäß Anspruch 2 oder 3, wobei die hervorstehenden Elemente (30) eines jeden Satzes hervorstehender Elemente zwischen ihnen entlang des Außengewindes (17) des zweiten Kupplungsabschnitts (15) ausgerichtet sind.

5. Kappe (100) gemäß einem der vorhergehenden Ansprüche, wobei die hervorstehenden Elemente (30) einen im Wesentlichen trapezförmigen oder rechteckigen Querschnitt aufweisen.

6. Kappe (100) gemäß einem der vorhergehenden Ansprüche, wobei die hervorstehenden Elemente (30) in Bezug auf eine Kante des Außengewindes (17) des zweiten Kupplungsabschnitts (15) vertieft oder ausgerichtet sind.

7. Kappe (100) gemäß einem der vorhergehenden Ansprüche, wobei die der Öffnung (11a) des axialen Hohlraums (11) zugewandte Oberfläche (22) des Gummis (20) eben oder konvex mit einer der Öffnung (11a) des axialen Hohlraums (11) zugewandten Konvexität ist.

8. Kappe (100) gemäß einem der vorhergehenden Ansprüche, wobei der axiale Hohlraum (11) eine Seitenoberfläche (12) und eine starre Bodenoberfläche (13) aufweist, an der eine Oberfläche (21) des Gummis (20) gegenüber der der Öffnung (11a) des axialen Hohlraums (11) zugewandten Fläche (22) anliegt.

9. Kappe (100) gemäß einem der vorhergehenden Ansprüche, wobei der Gummi (20) ferner mindestens eine Längsnut (24) aufweist, die in einer Seitenoberfläche (23) desselben ausgebildet ist.

10. Kappe (100) gemäß einem der vorhergehenden Ansprüche, wobei der zweite Kupplungsabschnitt (15) an einem freien Ende davon eine konische Öffnung (18) aufweist.

11. Spritze (1) mit Luer-Lock-Anschluss (5), bevorzugt eine vorgefüllte Spritze, umfassend eine Kappe (100) gemäß einem der vorhergehenden Ansprüche.

## Revendications

1. - Capuchon (100) pour une seringue (1) à raccord Luer (5) comprenant :
- un corps principal (10) comportant une première partie de préhension (14) et une seconde partie d'accouplement (15) ayant un filetage externe (17) pour un accouplement avec un filetage interne (6) correspondant du raccord Luer (5) de la seringue (1), une cavité axiale (11) ayant une ouverture (11a) à la seconde partie d'accouplement (15) étant formée dans le corps principal (10) ; et
- un élément en caoutchouc (20) reçu dans la cavité axiale (11) du corps principal (10), l'élément en caoutchouc (20) ayant une surface (22) faisant face à l'ouverture (11a) de la cavité axiale (11) et agencée pour venir en butée contre une extrémité libre ouverte (4a) d'un embout (4) de la seringue (1) pour fermer hermétiquement à l'air l'extrémité libre ouverte (4a) ;
**caractérisé par le fait que** des éléments surélevés (30) sont formés à des crêtes respectives de filets du filetage externe (17) de la seconde partie d'accouplement (15), et **par le fait que** les éléments surélevés (30) sont dimensionnés de façon à s'accoupler par frottement avec un fond (8) de filets correspondants du filetage interne (6) du raccord Luer (5).

2. - Capuchon (100) selon la revendication 1, dans lequel les éléments surélevés comprennent au moins deux ensembles d'éléments surélevés (30) agencés à des positions diamétralement opposées sur le filetage externe (17).

3. - Capuchon (100) selon la revendication 2, dans lequel chaque ensemble d'éléments surélevés comprend au moins un élément surélevé (30).

4. - Capuchon (100) selon la revendication 2 ou 3, dans lequel les éléments surélevés (30) de chaque ensemble d'éléments surélevés sont alignés entre eux le long du filetage externe (17) de la seconde partie d'accouplement (15).

5. - Capuchon (100) selon l'une quelconque des revendications précédentes, dans lequel les éléments surélevés (30) ont une section sensiblement trapézoïdale ou rectangulaire.

6. - Capuchon (100) selon l'une quelconque des revendications précédentes, dans lequel les éléments surélevés (30) sont en retrait ou alignés par rapport à un bord du filetage externe (17) de la seconde partie d'accouplement (15).

7. - Capuchon (100) selon l'une quelconque des revendications précédentes, dans lequel la surface (22) de l'élément en caoutchouc (20) faisant face à l'ouverture (11a) de la cavité axiale (11) est plane ou convexe avec une convexité faisant face à l'ouverture (11a) de la cavité axiale (11).

8. - Capuchon (100) selon l'une quelconque des revendications précédentes, dans lequel la cavité axiale (11) comprend une surface latérale (12) et une surface inférieure rigide (13), contre laquelle vient en butée une surface (21) de l'élément en caoutchouc (20) opposée à la surface (22) faisant face à l'ouverture (11a) de la cavité axiale (11).

9. - Capuchon (100) selon l'une quelconque des revendications précédentes, dans lequel l'élément en caoutchouc (20) comprend en outre au moins une rainure longitudinale (24) formée dans une surface latérale (23) de celui-ci.

10. - Capuchon (100) selon l'une quelconque des revendications précédentes, dans lequel la seconde partie d'accouplement (15) a une embouchure conique (18) à une extrémité libre de celle-ci.

11. - Seringue (1) à raccord Luer (5), de préférence une seringue préremplie, comprenant un capuchon (100) selon l'une quelconque des revendications précédentes.
